Europäisches Patentamt

**European Patent Office**

Office européen des brevets

(11) Veröffentlichungsnummer: **0 037 905**
**A1**

(12) **EUROPÄISCHE PATENTANMELDUNG**

(21) Anmeldenummer: **81101785.4**

(22) Anmeldetag: **11.03.81**

(51) Int. Cl.³: **G 01 N 1/30,** G 01 N 33/48

(30) Priorität: **16.04.80 DE 3014563**

(43) Veröffentlichungstag der Anmeldung: **21.10.81**
**Patentblatt 81/42**

(84) Benannte Vertragsstaaten: **BE CH DE FR GB IT LI NL SE**

(71) Anmelder: **Merck Patent Gesellschaft mit beschränkter Haftung, Frankfurter Strasse 250, D-6100 Darmstadt (DE)**

(72) Erfinder: **Fischer, Wolfgang, Dr., Kröhweg 27, D-6100 Darmstadt (DE)**
Erfinder: **Link, Renate, Rossbergring 63, D-6107 Reinheim 5 (DE)**

(54) **Verfahren zur Bestimmung leukämischer Zellen.**

(57) Die Erfindung betrifft ein Verfahren zum Nachweis und zur differentialdiagnostischen Bestimmung leukämischer Zellen und ist dadurch gekennzeichnet, daß man Leukozyten mit einem Supravitalfarbstoff behandelt und die unterschiedliche Färbung gesunder und leukämischer Zellen auswertet.

EP 0 037 905 A1

Merck Patent Gesellschaft
mit beschränkter Haftung
D a r m s t a d t


Verfahren zur Bestimmung leukämischer
Zellen


Die Erfindung betrifft ein Verfahren zum Nachweis und zur
differentialdiagnostischen Bestimmung leukämischer Zellen
durch selektive Anfärbung leukämischer Blutzellen.


Die bekannten Färbemethoden von Leukozyten sind unspezifisch,
weil normale und leukämische Zellen damit angefärbt werden.
Für eine Leukämiediagnose war man bisher darauf angewiesen,
durch Bestimmung der Anzahl von Leukozyten des in den Gefäßen zirkulierenden Blutes die pathogene Erhöhung derselben nachzuweisen. Die Leukämiediagnose über die Bestimmung veränderter enzymatischer Aktivitäten von Leukämiezellen ist zeitraubend und apparativ aufwendig. Aus Cell
13, 487 (1978) ist eine Färbemethode für leukämische Zellen
mit Hilfe von Merocyaninfarbstoffen bekannt, wobei die Auswertung fluoreszenzmikroskopisch erfolgt. Diese Methode
hat den Nachteil, daß ein Fluoreszenzmikroskop benötigt
wird, das erheblich teurer ist als ein normales Mikroskop
und deshalb auch nicht an allen Stellen vorhanden ist, an
denen die Durchführung eines Leukämietestes von Interesse
sein könnte. Ein weiterer Nachteil dieser Methode ist
darin zu sehen, daß gefärbte und ungefärbte Zellen nicht bei der gleichen Mikroskopeinstellung ausgewertet werden können, wodurch die Auswertung umständlich wird.

Der Erfindung liegt die Aufgabe zugrunde, ein einfaches Verfahren zur Verfügung zu stellen, mit dem leukämische Blutzellen durch spezifische Färbung von normalen Leukozyten unterschieden werden können.

Es wurde gefunden, daß man das unterschiedliche Verhalten von normalen Leukozyten und leukämischen Zellen gegenüber Supravitalfarbstoffen zu einer zuverlässigen Leukämiediagnose verwenden kann.

Gegenstand der Erfindung ist ein Verfahren zum Nachweis und zur differentialdiagnostischen Bestimmung leukämischer Zellen, das dadurch gekennzeichnet ist, daß man Leukozyten mit einem Supravitalfarbstoff behandelt und die unterschiedliche Färbung gesunder und leukämischer Zellen auswertet.

Bevorzugte Supravitalfarbstoffe zur Durchführung des erfindungsgemäßen Verfahrens sind Triphenylmethanfarbstoffe der Phtalein- bzw. Sulfophtalein-Klasse, wie Eosin, Erythrosin, Phloxin, Bromphenolblau, Bromchlorphenolblau und verwandte Farbstoffe.

Überraschenderweise hat sich gezeigt, daß sich mit diesen Farbstoffen differentialdiagnostisch einwandfreie Färbungen durchführen lassen, obwohl sich mit diesen Farbstoffen normalerweise eine Supravitalfärbung von Leukozyten überhaupt nicht durchführen läßt. Die gesunden Zellen bleiben deshalb auch ungefärbt, während die leukämischen Leukozyten angefärbt werden. Die Färbung tritt sehr schnell ein und die Farbunterschiede zwischen gesunden und kranken Zellen treten sehr deutlich hervor, so daß die Auswertung auch von angelernten Kräften

- 3 -                                    0037905

leicht durchgeführt werden kann. Mit Phloxin z.B. färben sich die leukämischen Zellen rot, mit Bromchlorphenolblau blau; die gesunden Zellen bleiben in beiden Fällen farblos.

Die Färbungen werden in an sich bekannter Weise durchgeführt, d.h. unter Bedingungen, unter denen die Supravitalfarbstoffe auch bisher zur Färbung der verschiedenen Blutbestandteile eingesetzt werden. Die Farbstofflösungen sollten in einer Konzentration von etwa 0,01 bis 0,5 %, vorzugsweise 0,05 bis 0,2 %, in physiologischer Kochsalzlösung vorliegen.

Zur Durchführung des Verfahrens wird EDTA-Blut z.B. mit einer Dextranlösung versetzt und im verschlossenen Röhrchen für eine Stunde bei 37$^{\circ}$C stehen gelassen. Der Plasmaüberstand wird abpipettiert, zentrifugiert und der Rückstand wird mit physiologischer Kochsalzlösung aufgenommen. Anschließend wird die Farbstofflösung hinzugeben und gemischt; ein Teil der Mischung wird auf einen Objektträger gebracht, sofort mit einem Deckglas abgedeckt und unter dem Mikroskop ausgewertet.

Die quantitative Auswertung wird so vorgenommen, daß man in einem bestimmten Gesichtsfeld des Durchlicht-Mikroskops die Zahl der gefärbten Leukozyten (x) sowie die Gesamtzahl der Leukozyten (y) feststellt und daraus den Prozentsatz (A) der gefärbten Zellen errechnet nach der Gleichung

$$A = \frac{x}{y} \cdot 100.$$

Zur Schnellbestimmung leukämischer Leukozyten ist es auch möglich, einige Tropfen des Plasmas auf den Objektträger aufzubringen und mit einer wasserlöslichen Folie abzudecken, die die erforderlichen Farbstoffe in homogener Verteilung enthält.

Die Erfindung wird durch die folgenden Beispiele näher erläutert.

Beispiel 1

Analyse von Blut mit verminderter, normaler und geringgradig erhöhter Leukozytenzahl (bis etwa 40.000/µl Blut)

2 ml EDTA-Blut wurden mit 8 Tropfen Dextranlösung (Dextran T 500, 6 %ige Lösung in 0,9 %iger Natriumchloridlösung) versetzt und im verschlossenen Röhrchen eine Stunde bei 37°C stehen gelassen. Anschließend wurde der Plasmaüberstand abpipettiert und 5 Min. lang bei 1000-2000 U/Min. zentrifugiert. Die überstehende Lösung wurde weggegossen, der Rückstand mit 2 Tropfen 0,9 %iger Natriumchloridlösung versetzt und gemischt. Zu dieser Lösung wurden 2 Tropfen einer 0,1 %igen Phloxinlösung in 0,85 %iger Natriumchloridlösung hinzugegeben und erneut gemischt. 0,02 ml dieser Mischung wurden auf einen Objektträger aufgebracht, sofort mit einem Deckglas abgedeckt und unter dem Durchlicht-Mikroskop ausgewertet (Vergrößerung: 6,3x1,25x25). Die gesunden Zellen waren farblos, die kranken Zellen rot gefärbt.

Beispiel 2

Analyse von Blut mit stark erhöhter Leukozytenzahl (ab etwa 40.000/µl Blut)

2 ml EDTA-Blut wurden mit 8 Tropfen Dextranlösung analog Beispiel 1 versetzt und im geschlossenen Röhrchen eine Stunde bei 37°C ruhig stehen gelassen. 0,02 ml des Plasmaüberstands wurden entnommen, mit 2 Tropfen Natriumchloridlösung versetzt und gemischt. Zu dieser Lösung wurden 2 Tropfen einer 0,08 %igen Bromchlorphenolblaulösung in 0,85 %iger Natriumchloridlösung hinzugegeben und erneut gemischt. 0,02 ml dieser Mischung wurden auf einen Objektträger aufgebracht, sofort mit einem Deckglas abgedeckt und unter dem Durchlicht-Mikroskop ausgewertet. Die gesunden Zellen waren farblos, die
kranken Zellen blau gefärbt.

Merck Patent Gesellschaft
mit beschränkter Haftung
D a r m s t a d t


Patentansprüche

1. Verfahren zum Nachweis und zur differentialdiagnostischen Bestimmung leukämischer Zellen,dadurch gekennzeichnet, daß man Leukozyten mit einem Supravitalfarbstoff behandelt und die unterschiedliche Färbung gesunder und leukämischer Zellen auswertet.

2. Verfahren nach Anspruch 1, dadurch gekennzeichnet, daß man einen Triphenylmethanfarbstoff aus der Phtalein- bzw. Sulfophtalein-Klasse verwendet.

# 0037905
Nummer der Anmeldung
EP 81 10 1785

**Europäisches Patentamt**

## EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| A | US - A - 4 131 800 (BRUCK et al.)<br><br>* Zusammenfassung; Spalte 5, Zeilen 40-57 *<br><br>--- | 1 |
| A | IBM TECHNICAL DISCLOSURE BULLETIN, Band 11, Nr. 3, August 1968, NEW YORK (US)<br>L.A. KAMENTSKY et al.: "Leukocyte assay", Seite 316<br><br>--- | 1 |
| A | BIOLOGICAL ABSTRACTS, Band 68, Nr. 7, Zusammenfassung 42491, Seite 4243,<br>J.S. HANKER et al.: "Facilitated Light microscopic cytochemical diagnosis of acute myelogenous leukemia"<br>& CANCER RES., 39(5), 1979, 1635-1639<br><br>--- | 1 |
| A | US - A - 3 906 120 (J.A. GEATING)<br><br>* Spalte 1, Zeile 21 bis Spalte 3, Zeile 29 *<br><br>--- | 1 |
| A | FR - A - 2 248 509 (COULTER ELECTRONICS INC.)<br><br>* Seite 1, Zeilen 1-31 *<br><br>& US - A - 3 893 767<br>& DE - A - 2 450 112<br><br>-------- | 1 |

**KLASSIFIKATION DER ANMELDUNG (Int. Cl.³)**

G 01 N 1/30
33/48

**RECHERCHIERTE SACHGEBIETE (Int. Cl.³)**

G 01 N 1/30
33/48

**KATEGORIE DER GENANNTEN DOKUMENTE**

X: von besonderer Bedeutung

A: technologischer Hintergrund

O: nichtschriftliche Offenbarung

P: Zwischenliteratur

T: der Erfindung zugrunde liegende Theorien oder Grundsätze

E: kollidierende Anmeldung

D: in der Anmeldung angeführtes Dokument

L: aus andern Gründen angeführtes Dokument

&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 17.07.1981 | KEMPIN |

EPA form 1503.1 06.78